# EUROPEAN PATENT SPECIFICATION

(11) **EP 0 413 248 B1**
(45) Date of publication and mention of the grant of the patent: **18.01.1995**
(21) Application number: 90115227.2
(22) Date of filing: 08.08.1990
(51) Int. Cl.: C08B 37/10, A61K 31/725

(54) **Substances with heparin-like structure and their method of production**
Substanzen mit heparinähnlicher Struktur und Verfahren zu deren Herstellung
Substances à structure ressemblant à celle de l'héparine et méthode de leur production

(30) Priority: 18.08.1989 AR 314703
(43) Date of publication of application: 20.02.1991
(73) Proprietor: AJORCA S.A., AR-1017 Buenos Aires (AR)
(72) Inventor: Fussi, Fernando, CH-1900 Lugano (CH); Diaz, Victor, Planta Baja - Buenos Aires (AR); Domanico, Ricardo, Villa Celina (1772), Buenos Aires (AR); Fuentes, Esteban, 7o piso "B", Buenos Aires (AR)
(74) Representative: Gervasi, Gemma, Dr.

(56) References cited:
- EP-A- 0 165 569
- WO-A-88/01280
- DE-A- 3 403 256
- BIOCHEMICAL JOURNAL, vol. 189, 1980, pages 625-633, London, GB; G. BENGTSSON etal.: "Interaction of lipoprotein lipase with native and modified heparin-like polysaccharides"
- CARBOHYDRATE RESEARCH, vol. 59, 1977, pages 285-288, Amsterdam, NL: S. HIRANOet al.: "Some N-acyl derivatives of N-desulphated heparin"

## Description

Natural glycosaminoglycans obtained by extraction, (GAG), such as the Heparansulfate (HS), have been isolated and characterized (Jorpes, J. - Gardell, S.JBC 176, 267-276). Their structure is similar to the Heparin one, varying fundamentally in the degree of N-acetylation: in the Heparin, the group -NH₂ of the glucosamine is preferably sulfated, instead, in the HS, is N-acetylated.

As a consequence of said structural difference, the HS shows peculiar biological characteristics, different from those of the Heparin:
1) It has low anticlottig activity tested in vitro (APTT, anti-X).
2) It shows profibrinolitic activity (release of the plasminogen tissular activator), when used in humans or in laboratory animals.
3) It presents a superior bioavailability to that of Heparin.

Therefore, said substances are specially relevant to cardiovascular medicine, mainly, in long-lasting therapies intended to prevent the formation of microthrombus, as well as to contribute to their dissolution. This can be achieved with little risk of hemorrhages and with reduced side effects.

Obviously, these natural mucopolysaccharides cannot be extracted in large scale, due to their low concentration in the tissular mass. This can limit significatively its extensive use in the therapeutical field.

Now, in accordance with the patent application, a process has been developed, which permits to obtain a pure N-acetylated compound with a Heparin-like structure with good yields, starting from Heparin as raw material.

The process of the invention is based upon a controlled hydrolysis of the Heparin with partial or complete N-desulfatation without depolymerization, followed by the introduction of the acetyl group in the specific position of the aminic N, through a reaction using acetic anhydride.

The Heparin desulfatation is achieved specifically on the amino group, without hydrolyzing the group O-sulfate and without producing hydrolysis of the glycosidic bond or other parasitic reactions. This has already been described by Inoue and Nagasawa (1975) "Inoue, Y. - Nagasawa, K. Carbohyd, Res. 46, 87-95 (1976)". However, this method cannot be easily conducted at large scale, since it works on a heparin-pyridinic complex dissolved in acqueous or methanolic dimethylsulfoxide with its associated problems, for example: the separation of the complex from the reacting solvent, the quantitative elimination of the pyridine, the recovery of expensive solvents, etc.

On the contrary starting from calcium Heparin, due to the contra-ion size, a selective N-desulfatation in an acid acqueous medium can be achieved, without causing structural alterations and without affecting the medium MW.

Particularly, the browning of the product and the hydrolysis of the O-sulfate group are avoided.

Then, the partially or completely N-desulfated Heparin (Intermediate A), is subjected to the acetylation reaction.

The Heparin acetylation has been described by Danishefsky (Danishefsky, N. Meth. Garb. Chem. 5, (85), 11) and Nagasawa as well (Inoue, Y. - Nagasawa, K. Carbohyd, Res. 46, 87-95 (1976)) and by EP-A-0 165 569. All these prior art processes work with great quantities of acetic anhydride, pH=6,5-7,0 and generally at low temperatures.

In these conditions, and working at low temperatures (0-5⁰C), a parasitic O-acetylation is produced over the free -OH of the disaccharidic unit.

It has been discovered, and it is the object of the present invention, that when using a quantity practically stoichiometric of the acetic anhydride (1-1,1 moles (CH₃CO)₂=O/Mol of the free-amino N, previously determined by the titration curve and at a pH between to 9,3 and 9,5 (maximum limit compatible with the acetic anhydride hydrolysis), the N-acetylation is exclusively produced, working at 25-30°C.

At this temperature, the reaction is fast and complete within 15 minutes. Even if the reaction is lasted for a longer time, it does not produce secondary reactions.

The obtained product (final product B) has an extremely constant composition and is represented by a molecule chemically well characterized, with a precise analytical description, as defined in the following table and figures.

To better identify the product object of the present invention the ¹³C-NMR characterisation of the product is hereby described.

The NMR spectrum of the final product B (figure 1), was determined in D₂O solution, at 75 MHz, by means of a Bruker Mod. CxP-300 spectrometer. For comparison purposes also the spectra of a standard Heparin (figure 2) ad of Heparansulfate (HS, figure 3) are enclosed; these spectra correspond to those known in the literature (B. Casu et al, Arzneim-Forsch. (Drug Research) 33, 135-142, 1983).

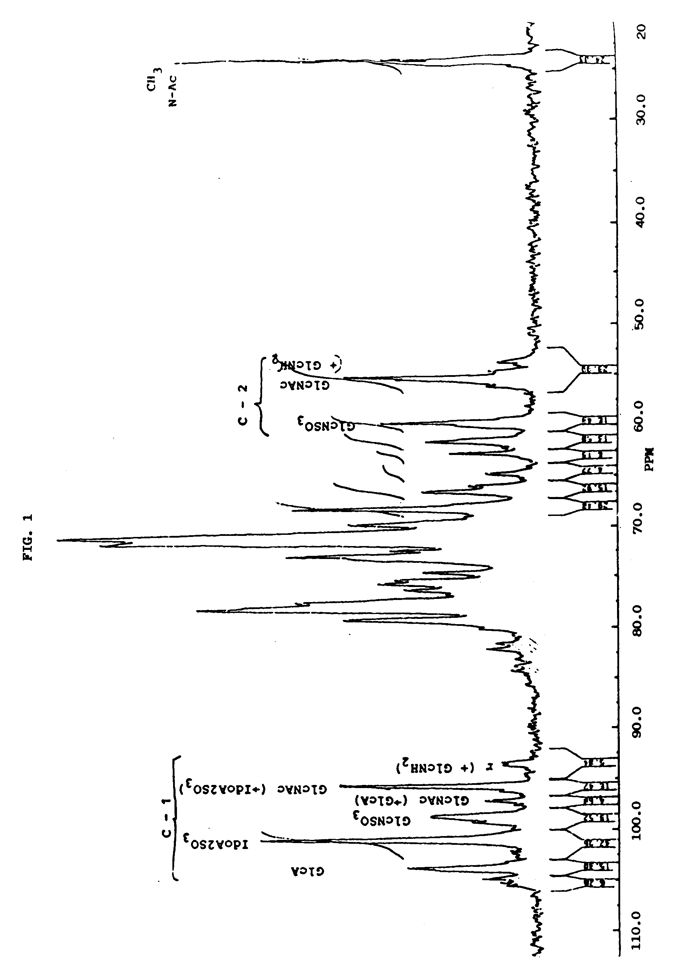

In figure 1, the peaks in the region 90-95 ppm (due to C-1 anomeric carbon) and in the region 53-61 ppm (due to C-2 of amino sugar units) are particularly suitable in order to identify the different uronic units, to measure the amount of said units and to determine the sulfate group distribution on the uronic and hexosamine units.

In the present text the following abbreviations have been adopted:
L-iduronyl-2-sulfate = Ido A2SO₃
D-glucuronic acid = GlcA
Glucosamine N-sulfate = GlcNSO₃
N-acetyl glucosamine = GlcAc
Glucosamine with no substituted amine groups = GlcNH₂
r = reduced terminal groups, mainly referred to hexosaminic units.

The following relative percentages were calculated by considering the areas under the characteristic peaks of different groups:
IdoA2SO₃ = 68.2%; GlcA = 31.8% referred to total uronic acids; GlcNSO₃ = 37.8%; GlcNAc bound to IdoA2SO₃=37.8%; GlcNAc bound to GlcA = 11.0%; reduced GlcN = 13.4%, with respect to total hexosamines (C-1 peaks evaluated).

From the area under C-2 and CH₃ (N-acetyl) peaks the GlcNSO₃ groups percentage amounts to 36.2%, and the percentage of total GlcNAc units amounts to 53%.

The product is analogous to Heparin (because of high IdoA2SO₃ unit content 71-91% of total uronic acid in Heparin) and to Heparansulfate (because of high N-acetyl group 58% content of total glucosamine Heparansulfate); but it differs in a considerable measure from abovementioned glycosamimoglycans, because of predominant GlcNAc→IdoA2SO₃ sequences which are in minor amount in both aforesaid GAG.

The N-acetylated derivative of heparin according to the invention has the following characteristics:
- absence of O-acetylated groups;
- anticoagulant activity (APTT Anti-X) of not more than 10 IU/mg;
- N-acetyl content (expressed as CH₃CO-) of 3 to 7% by weight corresponding to a degree of N-acetylation of from 40% to 90%;
- uronic acids content of 31% ± 1% by weight;
- hexosamines content of 32% ± 1% by weight;
- organic sulfate content (as S) of 8% ± 1% by weight;
- molar ratio of uronic/hexosamine/S/acetyl of 1/1/1.5-1.7/0.4-0.9; and
- specific optical rotation of greater than + 35°.

The example which follows, describes the original process for the production of Heparin-like N-acetylated compound (final product B) obtained from calcium Heparin. Based on laboratory models this compound shows fibrinolytic and antithrombotic activity, and lacks anticlotting activity. Therefore, the hemorrhagic risk is low. The antithrombotic activity has been tested using a model on a rat's tail, according to Bekemeyer (Bekemeyer H. - Hirschelmann, "Agents and Actions". Vol.16 pag. 446 (1985)).

The fibrinolytic activity ex-vivo was tested on the plasma of rabbits injected with the product through the evalutation of FDP (Fibrin Depolymerization Peptides), using the Boehringer Diagnostica Kit.

The method of production, as well as the complexes obtained and their use as fibrinolytic and protecting agents of thrombosis, are part of this invention.

The purification of the final product B and of the intermediate A are carried out following the general techniques, using ammonium quaternary salts and ethanol in saline solution, as described by Scott (Scott, J. Meth. Bioch. Analysis VIII 145-197 (1960)).

### EXAMPLE

### 1) Preparation of the calcium Heparin:

100 g of Heparin sodium 150 IU/mg are dissolved in distilled water, a quantity sufficient for 1 liter of solution (10%). It is heated at 50°C and, in agitation, 120 g of cetyltrimethylammonium bromide (CTAB) dissolved in 1 liter of distilled water, are slowly added.

The precipitate is left still for 1 hour at said temperature ad is separated by centrifugation. It is washed twice with 100 ml each time, of 1% solution of CTAB in water, and finally the solid substance obtained is dissolved in 1 liter of 2M CaCl₂ solution in water. Then, the polysaccharide is precipitated adding 1 volume of ethanol 96°. It is settled during all night, the supernatant is put aside and the solid substance obtained is redissolved in 1 liter of 2 M CaCl₂ in aqueous solution.

The clear solution is precipitated again with a volume of ethanol 96°. The solid substance is settled, redissolving for a second time in 1 liter 2 M CaCl₂. The rests of CTAB are eliminated by adding bentonite according to Scott ( Scott, J. Meth. Bioch. Analysis VIII, 145-197 (1960)). The bentonite is eliminated by filtration and the clear liquid is precipitated for the last time with 1 volume of ethanol.

The solid substance is anhydrated with ethanol and dried in a vacuum drying chamber.

Then, 90 g of calcium Heparin are obtained with a degree of sodium inferior to 0.1%.

### 2) Preparation of the intermediate A:

The 90 g of calcium polysaccharide are dissolved by flowing back in HCl 0.25 M, a quantity sufficient to obtain a 10% solution, heating at 80°C. After 2 hours, the N-desulfatation reaction is practically completed, which is evidenced by the absence of anticlotting activity of the liquid in reaction, and the appearance of a strong calcium sulfate precipitate.

When the action desired is completed, the reaction is stopped adjusting the pH to 7 by adding a 10 M NaOH solution.

A sufficient quantity of 10% w/v CO₃Na₂ solution is added to complete precipitation of CO₃Ca, and the precipitate obtained is eliminated by filtration.

The polysaccharide N-desulfated is isolated from the liquid adding 1 vol. ethanol and it is settled during the whole night. The heparaminic material is anhydrated and dried in vacuum drying stoves.

Then, 60 g of material are obtained containing 50% of free amino groups or the total N groups (Intermediate A).

### 3) Preparation of the final product B:

50 g of intermediate A are dissolved in distilled H₂O, a sufficient quantity for 1 liter of solution (5% w/v) and the pH is adjusted to 9,5 with 10 M NaOH solution. The temperature is raised to 25-28°C, and under agitation 5 ml of acetic anhydride are added drop by drop, maintaining the pH in 9,3 - 9,5 with a NaOH solution. The aggregate of acetic anhydride takes approximately 5 minutes and the reaction time under agitation is 30 minutes, after which 10 g of NaCl are added. Then, all the liquid is precipitated in 1 vol. ethanol and settled for the whole night.

After it has been precipitated and settled, the paste is dissolved in 500 ml of sterile water, 5 g of NaCl are added, the pH is adjusted to 6,0 with 6 M HCl and filtration is started using depyrogenic plates and sterilizing membranes of 0,8 - 0,22 U. The liquid filtered is precipitated with 1 vol. ethanol filtered, it is anhydrated and dried in vacuum drying stoves at 60°C, during 12 hours.

55 g of N-acetylated product are obtained, without free amino groups, but with a chemical composition (see titration curve), analytical characteristics, and biological and pharmacological properties similar as a whole to the ones of the natural HS.

The analytical tipification of the original Heparin, the intermediate A complex and the final product B are shown here below.

| Analytical Determ. | Sodium Heparin | Intermediate A | Final Prod. B |
|---|---|---|---|
| APPT Activity | 150 IU/mg | 10 IU/mg | 10 IU/mg |
| Anti-X Activity | 150 IU/mg | 10 IU/mg | 10 IU/mg |
| N-acetyl | 0,8% | 0,9% | 4,3% by W. |
| Uronic acid | 30,0% | 35,0% | 31,0% by W. |
| Hexosamines | 30,0% | 35,0% | 32,0% by W. |
| Organic sulfate (as S) | 11,0% | 8,2% | 7,8% by W. |
| Specific rotation | + 36° | + 40° | + 40° |
| Molar Relation uronic/hexosamine/S/acetyl | 1:1:2,2:0,1 | 1:1:1,7:0,1 | 1:1:1,7:0,5 |
| NH₂/N total | 0,0 | 0,5 | 0,0 |

The figures 1 to 3 are the ¹³C-NMR spectra of the following compounds:

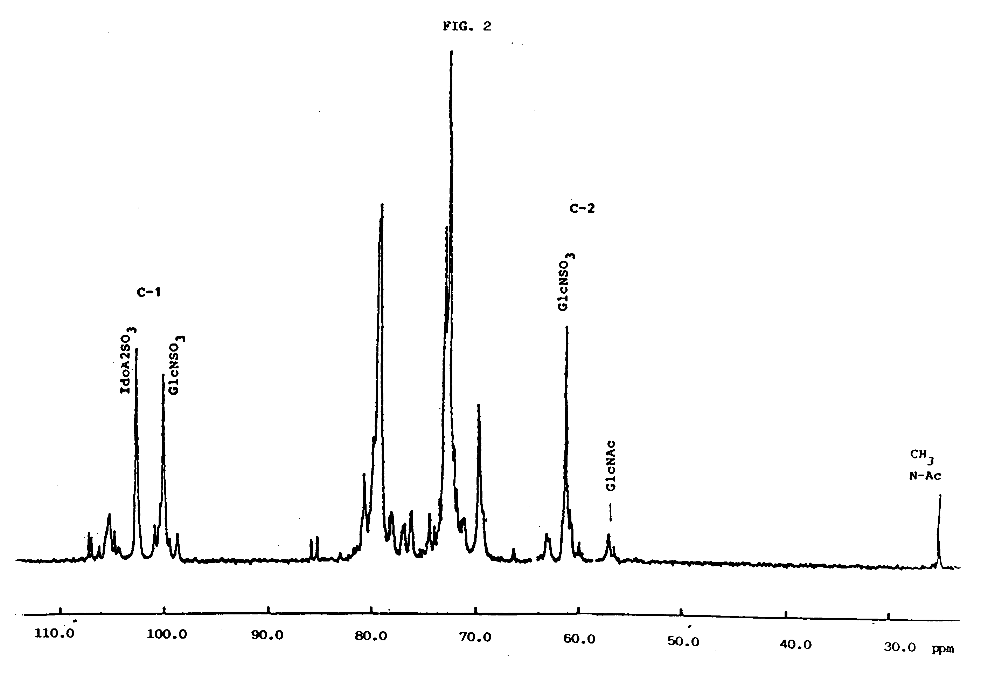

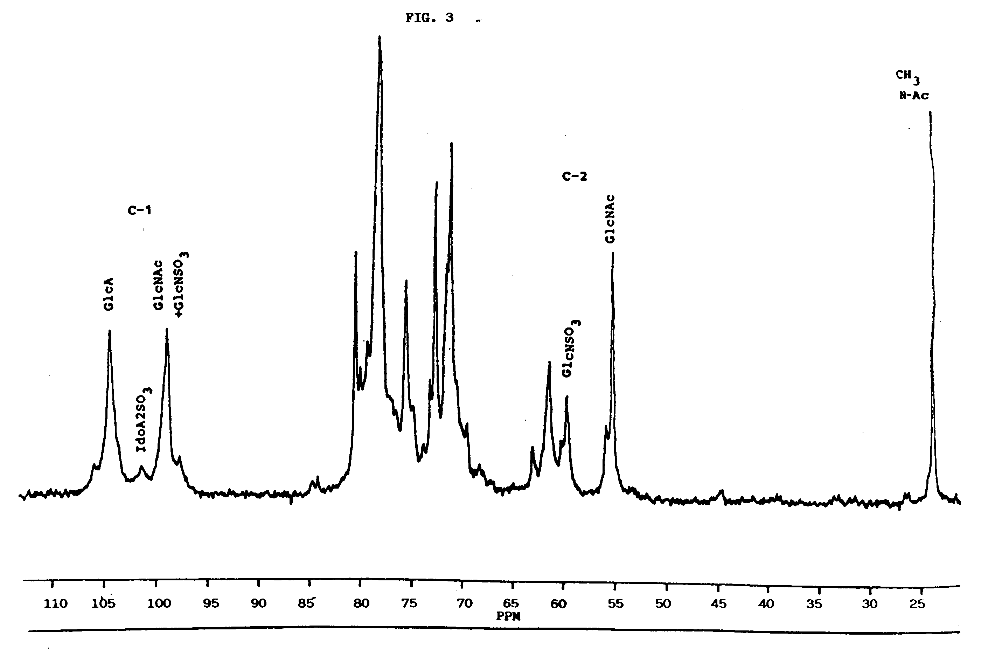

fig. 1: final product B; fig 2: Heparin; Fig. 3: Heparansulfate.

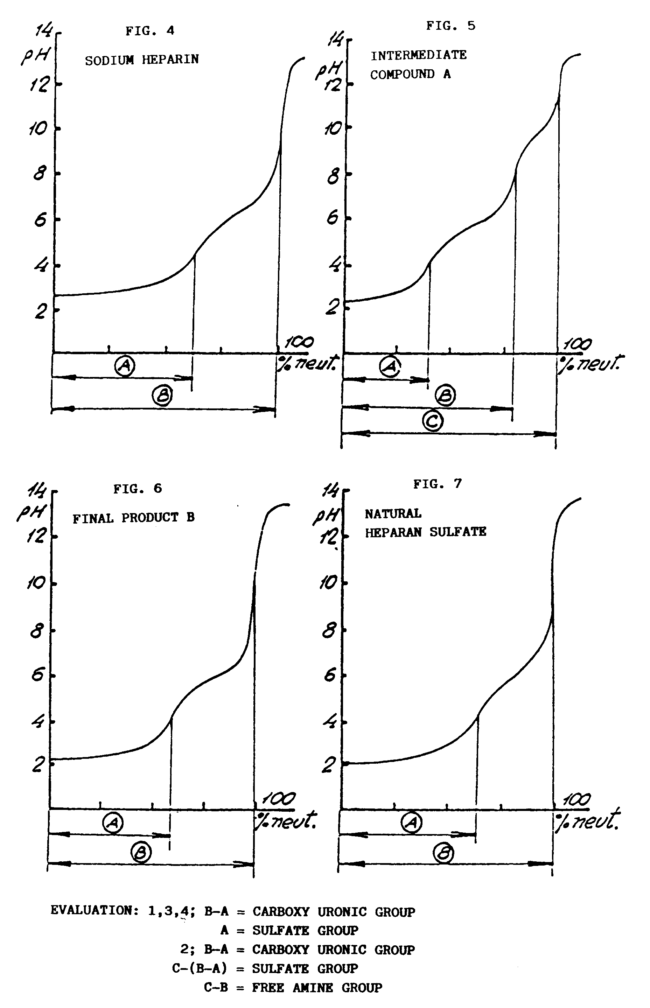

The figures 4 to 7 represent evalutations as per the following details:
- fig. 4,6,7:: B-A= carboxylic uronic groups
A= sulfate groups
- fig. 5 :: B-A= carboxylic uronic groups
C-(B-A)= sulfate groups
C-B= free amino groups

## Claims

1. A process for the synthesis of an N-acetylated compound starting from partially or completely N-desulfated heparins characterized in that per mol of free amino groups, as determined by titration, from 1 to 1.1 moles of acetic anhydride is added at a pH comprised between 9.3 and 9.5 and at a temperature between 25 and 30°C.

2. An N-acetylated derivative of heparin having the following characteristics:
- absence of O-acetylated groups;
- anticoagulant activity (APTT Anti-X) of not more than 10 IU/mg;
- N-acetyl content (expressed as CH₃CO-) of 3 to 7% by weight corresponding to a degree of N-acetylation of from 40% to 90%;
- uronic acids content of 31% ± 1% by weight;
- hexosamines content of 32% ± 1% by weight;
- organic sulfate content (as S) of 8% ± 1% by weight;
- molar ratio of uronic/hexosamine/S/acetyl of 1/1/1.5-1.7/0.4-0.9; and
- specific optical rotation of greater than +35°C.

3. A pharmaceutical composition comprising an effective amount of the product of claim 2 having antithrombic and fibrinolytic properties and low anticoagulant activity.

4. An N-acetylated derivative of heparin according to claim 2 wherein:
- N-acetyl content (expressed as CH₃-CO-) is 4.3% by weight;
- uronic acids content is 31.0% by weight;
- hexoxamines content is 32.0% by weight;
- anticoagulant activity: APTT Anti-X value is 10 IU/mg
- organic sulfate content (as S) is 7.8% by weight;
- molar ratio of uronic/hexosamine/S/acetyl of 1/1/1.7/0.5;
- specific optical rotation of 40°.

## Patentansprüche

1. Verfahren zur Synthese einer N-acetylierten Verbindung, ausgehend von teilweise oder vollständig N-desulfatierten Heparinen,
dadurch **gekennzeichnet**,
daß pro Mol freie Amino-Gruppen, wie bestimmt durch Titration, von 1 bis 1,1 Mole Essigsäureanhydrid bei einem pH zwischen 9,3 und 9,5 und bei einer Temperatur zwischen 25 und 30°C zugegeben werden.

2. N-acetyliertes Heparin-Derivat mit den folgenden Charakteristika:
- Abwesenheit von O-acetylierten Gruppen;
- Antigerinnungsaktivität (APTT Anti-X) von nicht mehr als 10 IU/mg;
- N-Acetyl-Gehalt (ausgedrückt als CH₃CO-) von 3 bis 7 Gew.%, entsprechend einem N-Acetylierungs-Grad von 40 bis 90 %;
- Uronsäuren-Gehalt von 31 ± 1 Gew.%;
- Hexosamine-Gehalt von 32 ± 1 Gew.%;
- organischer Sulfat-Gehalt (als S) von 8 ± 1 Gew.%;
- Molverhältnis von Uronsäure/Hexosamine/S/Acetyl von 1/1/1,5 - 1,7/0,4 - 0,9; und
- spezifische optische Drehung von mehr als +35°C.

3. Pharmazeutische Zusammensetzung, umfassend eine wirksame Menge des Produkts von Anspruch 2 mit antithrombotischen und fibrinolytischen Eigenschaften und einer niedrigen Antigerinnungsaktivität.

4. N-acetyliertes Heparin-Derivat gemäß Anspruch 2, worin:
- der N-Acetyl-Gehalt (ausgedrückt als CH₃-CO-) 4,3 Gew.% beträgt;
- der Uronsäuren-Gehalt 31,0 Gew.% beträgt;
- der Hexosamine-Gehalt 32,0 Gew.-% beträgt;
- die Antigerinnungsaktivität: APTT Anti-X-Wert 10 IU/mg beträgt;
- der organische Sulfat-Gehalt (als S) 7,8 Gew.% beträgt;
- das Molverhältnis von Uronsäuren/Hexosamine/S/Acetyl 1/1/1,7/0,5 beträgt;
- die spezifische optische Drehung 40° beträgt.

## Revendications

1. Procédé pour synthétiser un composé N-acétylé, en partant d'héparines partiellement ou complètement N-désulfatées, caractérisé en ce que, par mole de groupes amino libre, dont la quantité est déterminée par titrage, on ajoute de 1 à 1,1 modes d'anhydride acétique à un pH compris entre 9,3 et 9,5 et à une température comprise entre 25 et 30°C.

2. Dérivé N-acétylé de l'héparine, présentant les caractéristiques suivantes :
- absence de groupes O-acétylés ;
- activité anticoagulante (APTT anti-X) non supérieure à 10 UI/mg ;
- teneur en N-acétyle (exprimée en CH₃CO-) de 3 à 7 % en poids, ce qui correspond à un degré de N-acétylation de 40 à 90 % ;
- teneur en acides uroniques de 31 ± 1 % en poids ;
- teneur en hexosamines de 32 ± 1 % en poids ;
- teneur en sulfate organique (sous forme de S) de 8 ± 1 % en poids ;
- rapport molaire uronique/hexosamine/S/acétyle égal à 1/1/1,5-1,7/0,4-0,9 ; et
- pouvoir rotatoire spécifique supérieur à +35°.

3. Composition pharmaceutique comprenant une quantité efficace du produit selon la revendication 2, présentant des propriétés antithrombotiques et fibrinolytiques, ainsi qu'une faible activité anticoagulante.

4. Dérivé N-acétydé de l'héparine selon la revendication 2, dans lequel :
- la teneur en N-acétyle (exprimée sous forme de CH₃-CO-) est de 4,3 % en poids ;
- la teneur en acides uroniques est de 31,0 % en poids ;
- la teneur en hexosamines est de 32,0 % en poids ;
- activité anticoagulante : la valeur de l'indice APTT anti-X est de 10 UI/mg ;
- la teneur en sulfate organique (sous forme de S) est de 7,8 % en poids ;
- le rapport molaire uronique/hexosamine/S/acétyle est de 1/1/1,7/0,5 ;
- le pouvoir rotatoire spécifique est de 40°.
